# EUROPÄISCHE PATENTSCHRIFT

(11) **EP 3 924 186 B1**
(45) Veröffentlichungstag und Bekanntmachung des Hinweises auf die Patenterteilung: **02.12.2026**
(21) Anmeldenummer: 20712471.0
(22) Anmeldetag: 10.03.2020
(51) Int. Cl.: B33Y 40/00, A61C 13/34, A61K 6/62, A61K 6/887, B33Y 70/00, B33Y 80/00, C08F 2/48, C08F 220/30, C08F 290/06, C08K 3/013, C08L 33/08, C08L 33/10

(54) **STRAHLENHÄRTBARE ZUSAMMENSETZUNG ZUR VERWENDUNG IN RAPID-PROTOTYPING- ODER RAPID-MANUFACTURING-VERFAHREN**
RADIATION-CURABLE COMPOSITION FOR USE IN RAPID PROTOTYPING OR RAPID MANUFACTURING METHODS
COMPOSITION DURCISSABLE PAR RAYONNEMENTS DESTINÉE À ÊTRE UTILISÉE DANS UN PROCÉDÉ DE PROTOTYPAGE RAPIDE OU DE FABRICATION RAPIDE

(30) Priorität: 11.03.2019 DE 102019106151
(43) Veröffentlichungstag der Anmeldung: 22.12.2021
(73) Patentinhaber: Kulzer GmbH, 63450 Hanau (DE)
(72) Erfinder: HOHMANN, Alfred, 61389 Schmitten (DE)
(74) Vertreter: Bendele, Tanja
(86) Internationale Anmeldenummer: PCT/EP2020/056354
(87) Internationale Veröffentlichungsnummer: WO 2020/182814

(56) Entgegenhaltungen:
- EP-A1- 2 986 654
- EP-A1- 3 632 995
- US-A1- 2014 131 908
- US-A1- 2016 332 367
- US-A1- 2019 053 883

## Beschreibung

Gegenstand der Erfindung ist eine polymerisierbare, strahlenhärtbare, insbesondere UV/Vis-, UV- oder Vis-härtbare, Zusammensetzung umfassend (i) Monomere und (ii) mindestens eine weitere Komponente nach Anspruch 1. Des Weiteren ist Gegenstand der Erfindung ein Rohling in Form eines dreidimensionalen Formkörpers einer polymerisierten Zusammensetzung, insbesondere einer strahlengehärteten Zusammensetzung zur Herstellung dentaler prothetischer Teile, orthopädischer Apparate oder dentaler Pre-Forms als auch die Verwendung der Zusammensetzung zur Herstellung dentaler prothetischer Teile, orthopädischer Apparate oder dentaler Pre-Forms in einem Rapid-Prototyping- oder in einem Rapid-Manufacturing- oder Rapid-Tooling-Verfahren.

Neben handwerklichen Fertigungsmethoden gewinnen digitale Fertigungsmethoden wie subtraktive und additive Verfahren (Material-aufbauende Verfahren) im Dentalbereich immer mehr an Bedeutung. Der Vorteil bei additiven Verfahren liegt in der Einsparung von teuren Rohstoffen sowie einer schnelleren Fertigung der Objekte. Generative Verfahren sind im Dentalbereich z.B. schon in Form des Lasersinterns aus CoCr, Ti oder Polymeren zur Herstellung von Kronen und Brücken, Implantatbauteilen oder Modellen bekannt.

Zusammensetzungen von Acrylaten oder Derivaten von Acrylaten zur Herstellung von Zahnersatz mit entsprechendem Eigenschaftsprofil in Bezug auf die mechanischen Anforderungen im Dentalbereich nach der DIN EN ISO 20795-2 sind bislang nicht erhältlich (siehe Quintessenz Zahntechnik 2017-43(10): Seite 1325). Daher besteht grundsätzlich ein Bedarf an Materialien für die Herstellung von anatomischen Modellen, anatomischen Tischmodellen und insbesondere zur Herstellung anatomischer Modelle als Ersatz des dentalen Gipsmodells, das aus einer Abformung der Zahnsituation und der Gingiva bzw. des Gebisses eines Patienten hergestellt wird, oder zur Herstellung prothetischer Teile oder definitivem Zahnersatz. Ferner besteht somit auch ein Bedarf an Zusammensetzungen zur Herstellung von definitiven prothetischen Teilen, orthopädischer Apparate oder dentalen Pre-Forms.

Die Abformung und das Modell, hier auch anatomisches Modell oder Arbeitsmodell genannt, bilden die Basis für passgenauen Zahnersatz. Nur wenn Zahnarzt und Zahntechniker die materialspezifischen Anforderungen bei Abformung und Modellherstellung einhalten, erhalten sie eine individuelle optimale Versorgung.

Zur Modellherstellung verwendet der Zahntechniker in der Regel Gips. Er ist einfach anzuwenden und erfüllt die Anforderungen an präzise, dimensionsgetreue, Oberflächen glatte Modelle. Nachfolgend werden die Anforderungen an ein dentales, anatomisches Gipsmodell wiedergegeben: Volumenstabilität, geringste Expansion, keine Kontraktion, Lagerungsstabilität, kompatibel mit Desinfektionsmitteln, Isoliermitteln und Wachsen, eine glatte und porenfreie Oberfläche, ausreichende Druckfestigkeit, hohe Kantenstabilität, gute Abrasionsbeständigkeit, hohe thermische Belastbarkeit, insbesondere beim Ausbrühen von Wachs, in Verbindung mit und ohne Wasser.

EP3632995A1 betrifft dielektrische Tintenzusammensetzung. EP 2986654A1 offenbart strahlenhärtbare Zusammensetzungen umfassend u.a. aliphatische Urethan(meth)acrylat-Oligomere. US 2014/0131908A1 offenbart polymerisierbare Zusammensetzung umfassend Polymere, MMA sowie weitere Monomere. US 2016/0332367 A1 offenbart Zusammensetzungen umfassend Oligomere, Tris(2-hydroxyethyl)isocyanuratriacrylat, TEGDMA und Dentalglas. US 2019/053883 A1 offenbart ebenfalls Tris(2-hydroxyethyl)isocyanuratriacrylat und TEGDMA enthaltende Zusammensetzungen.

Aufgabe der vorliegenden Erfindung war es, eine Zusammensetzung bereitzustellen, die gute strahlenhärtbare Eigenschaften, insbesondere UV- und/oder Vis-Strahlen härtbare Eigenschaften, mit guter Polymerisationstiefe bei der Strahlenhärtung aufweist. Des Weiteren sollten die strahlengehärteten Zusammensetzungen sowohl bei Raumtemperatur als auch bei erhöhter Temperatur gute mechanische Eigenschaften aufweisen, um diese zur Herstellung von anatomischen Modellen einsetzen zu können. Die Zusammensetzungen sollen daher die Anforderungen von Modell-Materialen, zur Herstellung von Modellen für Modellguss, Implantologie, Sägeschnitt und Meistermodellen sowie präzisen Gegenbissmodellen erfüllen. Es bestand daher auch die Aufgabe lichthärtende, in additiven-/generativen Verfahren verwendbare Zusammensetzungen bereitzustellen, die als gedruckter Formkörper im gehärteten Zustand die folgenden Anforderungen erfüllen: Beibehaltung der mechanischen Eigenschaften und Dimensionsstabiliät bei Temperaturen von 45 °C bis 55 °C im Drucktopf, Dimensionsstabilität im Tiefziehprozess, sowie auch bei einer Reinigung bspw. mit einem Dampfstrahler, Lagerstabilität der lichthärtenden Zusammensetzung ohne signifikante Viskositätsänderung über den Lagerzeitraum, ausreichende Reaktivität bei Belichtung mit Laser, LED oder DLP-Projektor, Werkstücke/Formkörper mit ausreichender geometrischer Präzision/Auflösung druckbar, Farbstabilität der Mischung, und keine oder nur geringe Thixotropie. Aufgabe der Erfindung war es daher eine strahlenhärtbare Zusammensetzung bereitzustellen, die polymerisiert bei einer Prüfung in Wasser bei 55 °C eine Biegefestigkeit von mindestens 40 MPa und ein Biegemodul von mindestens 800 MPa aufweist.

Nachfolgend sind die weiteren Anforderungen an ein dentales Gipsmodell in verschiedenen Arbeitsschritten skizziert: Modell in Verbindung mit thermischen Einflüssen und Wasser: Angießen von Kunststoffsattel bei Modellguss (20 Min. bei 55 °C im Wasserbad), Fertigstellung einer Teilprothese/Modellguss (20-30 Min. bei 55 °C im Wasserbad), Fertigstellung einer Totalprothese (Injektionstechnik Palajet/Küvetten Technik - 30 Min. bei 55 °C im Wasserbad), Fertigstellung einer Totalprothese Stopf-Presstechnik (30 - 40 Min. bei 100 °C im Wasserbad), Tiefziehschienen (20 - 30 Min. bei 55 °C), Wasserdampf zur Reinigung (70 °C - 110 °C), Abbrühen (3 - 5 Min. bei 80 °C - 100 °C), Verblendung mit Pala Veneers (55 °C, 2 bar, 20 Min.).

Modell in Verbindung mit Isolierungen und mechanischen Einflüssen: Ein-Artikulieren, d.h. das Modell wird im Artikulator mechanisch belastet, Isolierung gegen Prothesenkunststoff, Isolierung gegen Verblendkomposit, Isolierung gegen Wachs, Ausblockbarkeit mit UV-Kunststoff und/oder Wachs, Tauchwachs.

Modell in Verbindung mit thermischen Einflüssen ohne Wasser: Tiefziehen (155 - 170 °C/1 bis 2 Minuten), Heißklebepistole zum Fixieren der Modelle, Klebewachs zum Fixieren der Modelle, Ausblockbarkeit mit UV-Kunststoff und/oder Wachs, Tauchwachs, Tiefziehfolie und manuelle Härtung von Kompositen in Hi-Lite Power/3D (zweimal je 90 Sek. und einmal 180 Sek.), Härtung von Aufbissschienen in Hi-Lite Power/3D (zweimal je 5 Min.), Frässtumpf für Frästechnik (Temperatur-beständig gegen Stauhitze).

Verhalten der polymerisierten Zusammensetzung bei anderen Arbeitsschritten: Dimensionsstabil und gute Reinigungseigenschaften im Ultraschallbad mit Isopropanol, Verfärbung und Reinigung bei Artikulationsfolie (Bausch, rot, blau, schwarz), Verfärbung und Reinigung bei Okklusionsspray (verschiedene Hersteller), Abriebfestigkeit an der Präparationsgrenze (Kronen, Brücken, Modellguss-Klammerzähne), sprung/abriebfest (im Artikulator - Gips gegen Kunststoff; Kunststoff gegen Kunststoff).

Situationsmodelle sowie alle Arbeitsmodelle stellen die Grundlage für alle weiteren zahntechnischen Arbeiten dar. Zur Erfüllung hoher ästhetischer Ansprüche müssen Harz-Mischungen für den definitiven Zahnersatz, wie z.B. Arbeitsmodelle, KFO Modelle Dimensionsstabilität und Thermostabilität aufweisen. Möchte man Gipsmodelle durch gedruckte Kunststoffmodelle ersetzen um die Vorteile des digitalen Workflows zu erhalten, müssen die Kunststoffmodelle die gleichen positiven Eigenschaften wie Gipse aufweisen. Aufgrund verschiedener physikalischer und chemischer Randbedingungen unterliegen Kunststoffe aber einer thermischen Plastizität, die es gilt durch eine optimale Auswahl der Monomere in Grenzen zu halten.

Bisher gibt es keine Materialien, die alle genannten thermostabilen Eigenschaften aufweisen, so dass diese nur für einige zahntechnische Anwendungen verwendbar sind.

Der Erfindung lag daher ebenfalls die Aufgabe zu Grunde eine Zusammensetzung umfassend Monomere bereitzustellen, die eine besonders hohe Thermostabilität und Dimensionstreue aufweist, um bei Verwendung der polymerisierten Zusammensetzung als Formteil im Drucktopf sowie beim Tiefziehen von Aufbissschienen den erhöhten Temperaturen standzuhalten. Bei der Herstellung von Alignern muss mehrfach den höheren Temperaturen standgehalten werden.

Gelöst werden die Aufgaben der Erfindung durch die Zusammensetzung nach Anspruch 1 sowie die polymerisierte Zusammensetzung nach Anspruch 10 als auch den Rohling nach Anspruch 11 und die Verwendung nach Anspruch 12. Bevorzugte Ausführungsformen werden in den Unteransprüchen und detailliert in der Beschreibung offenbart.

Gegenstand der Erfindung sind polymerisierbare Zusammensetzungen, insbesondere strahlenhärtbare, vorzugsweise mittels UV/VIS-, UV- oder VIS-Strahlung polymerisierbare Zusammensetzungen, umfassend (i) Monomere und (ii) mindestens eine weitere Komponente nach Anspruch 1.

Die (i) die Monomere umfassen (a.1) mindestens ein aus 1,3,5-Tris(ω-hydroxyalkyl)-isocyanurat abgeleitetes Triacrylat, wobei der Hydroxyalkyl-Rest jeweils unabhängig 1 bis 8 C-Atome umfasst, und insbesondere mit 3 bis 8 C-Atomen linear, verzweigt und/oder cyclisch ist, bevorzugt ist ein Hydroxyalkyl-Rest mit 1 bis 6 C-Atomen, besonders bevorzugt mit 1 bis 4 C-Atomen, vorzugsweise mit 1 bis 3 C-Atomen, besonders bevorzugt ist Hydroxyethyl, und (a.2) mindestens ein di-funktionelles Monomer, das kein Urethanacrylat oder Urethanmethacrylat ist, und (ii) die mindestens eine weitere Komponente umfasst mindestens einen Photoinitiator für den UV- und/oder Vis-Bereich oder ein Photoinitiatorsystem für den UV- und/oder Vis-Bereich sowie optional mindestens einen Stabilisator, und optional weitere übliche Additive, optional Pigment(e) oder Farbstoff(e).

Besonders bevorzugte Zusammensetzungen weisen eine Viskosität bei Raumtemperatur (ca. 20 °C bis 23 °C) von kleiner gleich 5000 m·Pas, bevorzugt von kleiner gleich 3000 m·Pas, besonders bevorzugt von 500 bis kleiner 2500 m·Pas auf.

Nach einer Ausführungsform umfassen bevorzugte Monomere (a.1) mindestens ein aus 1,3,5-Tris(ω-hydroxyalkyl)isocyanurat abgeleitetes Triacrylat, besonders bevorzugt sind 1,3,5-Tris(2-hydroxyethyl)isocyanurat-Triacrylat, 1,3,5-Tris(2-hydroxy-methyl)isocyanurat-Triacrylat, 1,3,5-Tris(2-hydroxyethyl)-isocyanurat-Trimethacrylat, 1,3,5-Tris(2-hydroxymethyl)isocyanurat-Trimethacrylat oder Mischungen enthaltend mindestens zwei der Monomere.

Weitere bevorzugte (i) Monomere sind ausgewählt aus: (a.2) mindestens einem di-funktionellen Monomer, das kein Urethan(meth)acrylat ist, welches umfasst (b) und/oder (c), mit
(b) difunktionelle Acrylate mit bivalenter alicyclischer Gruppe und difunktionelle Methacrylate mit bivalenter alicyclischer Gruppe, und optional
(c) mindestens ein disubstituiertes 4,4'-Di(oxabenzol)dialkylmethan der Formel I 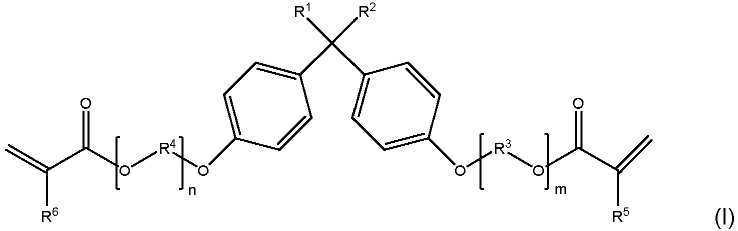
   mit R¹, R², R⁵ und R⁶ jeweils unabhängig ausgewählt aus H oder C1 bis C4-Alkyl, und mit
   R³ und R⁴ jeweils bivalent C1 bis C4-Alkylen, mit n = 0 bis 6 und m = 0 bis 6, und (d) mindestens ein mindestens difunktionelles Urethan(meth)acrylat,
   (e) mindestens ein monofunktionelles Acrylat mit alicyclischer Gruppe und/oder mono-funktionelles Methacrylat mit alicyclischer Gruppe und optional umfassend die Monomere die Monomere (f).

Bevorzugt sind disubstituierte 4,4'-Di(oxabenzol)dialkylmethane der Formel I mit R¹ und R² jeweils Methyl-, und R⁵ und R⁶ gleich und ausgewählt aus H, Methyl und Ethyl, insbesondere mit R⁵ und R⁶ gleich und ausgewählt aus H und Methyl, und mit R³ und R⁴ jeweils unabhängig bivalent Ethylen oder Propylen mit n = 1 bis 6, bevorzugt n = 2 bis 4, und mit m = 1 bis 6, bevorzugt m = 2 bis 4, bevorzugt mit n = 2 und m = 2 oder mit n = 4 und m = 4 sowie Mischungen dieser.

In bevorzugten Alternativen können in der Formel I R¹ und R² jeweils Methyl-, und R⁵ und R⁶ gleich und ausgewählt sein aus H, Methyl und Ethyl, bevorzugt sind R⁵ und R⁶ gleich und ausgewählt aus H und Methyl, und mit R³ und R⁴ jeweils unabhängig bivalent Ethylen oder Propylen mit n = 1 bis 6, wobei bevorzugt n = 2 bis 4, und mit m = 1 bis 6 sind, besonders bevorzugt n = 2 bis 4 und m = 2 bis 4, weiter bevorzugt mit n = 2 und m = 2 oder mit n = 4 und m = 4 sowie Mischungen dieser. Besonders bevorzugt ist eine Mischung von 4,4'-Di(oxabenzol)dialkylmethan der Formel I a) mit R¹ und R² jeweils Methyl-, und R⁵ und R⁶ gleich H und mit R³ und R⁴ jeweils unabhängig bivalent Ethylen mit n = 1 bis 6, bevorzugt n = 2 bis 4, und mit m = 1 bis 6, bevorzugt m = 2 bis 4, bevorzugt mit n = 4 und m = 4 sowie Mischungen dieser in Mischungen mit b) mit R¹ und R² jeweils Methyl-, und R⁵ und R⁶ gleich Methyl und mit R³ und R⁴ jeweils unabhängig bivalent Ethylen mit n = 1 bis 6, bevorzugt n = 2 bis 4, und mit m = 1 bis 6, bevorzugt m = 2 bis 4, bevorzugt mit n = 2 und m = 2 sowie Mischungen dieser.

Ein bevorzugtes mindestens di-funktionelles Monomer, das kein Urethan(meth)acrylat ist, ist ausgewählt aus (b) difunktionellen Acrylaten mit bivalenter alicyclischer Gruppe und difunktionellen Methacrylaten mit bivalenter alicyclischer Gruppe. Besonders bevorzugt sind (b) sie ausgewählt aus Tricyclodecandimethanoldiacrylat (TCDDA), Tricyclodecan-dimethanoldimethacrylat, Tricyclodecandiethanoldiacrylat, Tricyclodecandiethanoldimethacrylat und/oder Mischungen dieser (Teils synonym zu Bis(methacryloyloxymethyl)-tetrahydrodicyclopentadien oder Bis(acryloyloxymethyl)tetrahydrodicyclopentadien).

Vorzugsweise weisen alle erfindungsgemäßen Monomere (a), (b), (c), (d), (e) und (f) ein mittleres Molekulargewicht (Gewichtmittel) von kleiner 2.000 g/mol auf, besonders bevorzugt weisen die Monomere (a), (b), (d), (e) und (f) ein mittleres Molekulargewicht von kleiner 1000 g/mol auf.

Teilkristalline Kunststoffe (viele gebräuchliche Kunststoffe weisen einen kristallinen Anteil von 10 bis 80 % auf) besitzen sowohl eine Glasübergangstemperatur, unterhalb derer die amorphe Phase einfriert (einhergehend mit Versprödung), als auch eine Schmelztemperatur, bei der sich die kristalline Phase auflöst. Ein Glasübergang ist kein Phasenübergang 1. Ordnung und daher auch nicht mit einer exakten Temperatur verknüpft wie der Schmelzpunkt bei Kristallen. Je nachdem, auf welcher Zeit- und Längenskala bzw. Bewegungsmodus der molekularen Dynamik die verwendete Messmethode (s. u.) empfindlich ist, variiert der gefundene Wert systematisch. Ob ein Kunststoff oberhalb oder unterhalb seiner Glasübergangstemperatur verwendet werden kann, hängt von der Art des Kunststoffs ab (dabei ist zu beachten, dass die Glasübergangstemperatur eines Kunststoffes mit seiner Vernetzungsdichte steigt, d. h. die Glasübergangstemperatur eines Duroplasts ist deutlich höher als die eines Thermoplasts).

Auch bei der Auswahl der Monomere ist darauf zu achten, dass diese sich mit dem optional eingesetzten Füllstoff gut verbinden. In der Regel gehen Polyurethane, Acrylate, Polyester und andere Monomere keinen guten Verbund mit den eingesetzten Füllstoffen ein. Daher werden in der Regel die Füllstoffe für eine verbesserte Anbindung mit den Monomeren an den Oberflächen silanisiert oder hydrophobiert.

Sofern aufgrund der spezifischen dentalen Anwendung in der polymerisierbaren Zusammensetzung keine anorganischen Füllstoffe, bspw. aufgrund der angestrebten Viskosität der Zusammensetzung, eingesetzt werden können, besteht die Möglichkeit zur Reflexion der Strahlung, insbesondere diffusen Reflexion bzw. Streuung der eingestrahlten Strahlung, Farbstoffe oder Pigmente in der Zusammensetzung einzusetzen. Als Farbstoffe gelten Verbindungen, die in der polymerisierbaren Zusammensetzung löslich sind und vorzugsweise eine klare Lösung bilden.

Die erfindungsgemäßen, strahlenhärtbaren Zusammensetzungen können vorzugsweise mit einer Strahlenquelle die Licht im Vis-Bereich emittiert bestrahlt werden, besonders bevorzugt sind Strahlenquellen die von 360 bis 750 nm Strahlung emittieren, insbesondere bei ca. 385 nm, besonders bevorzugt bei ca. 405 nm. Besonders bevorzugt kann die erfindungsgemäße Zusammensetzung mit einer polychromatischen Strahlenquelle, wie einem DLP-Projektor, oder vorzugsweise mit einer monochromatischen Strahlenquelle, wie einem Laser-Projektor, im Vis-Bereich von 380 bis 660 nm bestrahlt werden.

Bei Zusatz dieser Pigmente und/oder Farbstoffe kann in der Zusammensetzung der Gehalt an Photoinitiator vermindert werden. Ein zu hoher Gehalt an Photoinitiator kann zu einem sogenannten "Overcuring" der bestrahlten Zusammensetzung Ungenauigkeiten und/oder Geometrieveränderung führen, so dass die entsprechend hergestellten dentalen Teile nicht verwendbar sind.

Der Einsatz der optional verwendbaren erfindungsgemäßen anorganischen Füllstoffe, Pigmente oder Farbstoffe führt zu einer gleichmäßigen Streuung der Strahlenquellen, insbesondere der UV- und Vis-Strahlenquelle in der Monomermatrix der Zusammensetzung, so dass eine gleichmäßigere Härtung der Zusammensetzung angenommen wird. Im Ergebnis weisen die polymerisierten Zusammensetzungen erhöhte Werte bei der erzielten Brucharbeit auf.

Die erfindungsgemäße Zusammensetzung weist nach einem Belichten mit einer Strahlenquelle im Vis-Bereich, insbesondere von 385 bis 405 nm, bevorzugt nach dem Belichten in einem Stereolithographie-Verfahren und Erhalten einer polymerisierten Zusammensetzung, vorzugsweise in Form eines Rohlings, 3D-Formteils, dentalen prothetischen Teils, anatomischen Modells, anatomischen Tischmodells, dentalen Arbeitsmodells, dentalen Vollmodells, dentalen Stumpfmodells, anatomischen oder dentalen Sägeschnittmodells, insbesondere Situationsmodells, Gegenbissmodells, Funktionsmodells, Vormodells, Reparaturmodells, Präzisionsmodells, Meistermodells sowie präzisen Gegenbissmodells, anatomischen Modells zur Ersetzung des dentalen Gipsmodells des Gebisses, prothetischer Teile, orthopädischer Apparate oder dentaler Pre-Form, sowie optionaler Nachvergütung der polymerisierten Zusammensetzung mit einer Strahlenquelle, die folgenden Eigenschaften auf a) eine Biegefestigkeit von größer gleich 40 MPa, insbesondere größer gleich 75 MPa und/oder b) ein E-Modul von größer gleich 800 MPa, insbesondere größer 1500 MPa, insbesondere größer gleich 2000 MPa in Anlehnung an DIN EN ISO 20795-2, insbesondere in der Regel bei Raumtemperatur, vorzugsweise 23 °C plus/minus 2 °C, bevorzugt von Raumtemperatur bis (in Wasser) 55 °C. Vorzugsweise weist die strahlengehärtete Zusammensetzung, insbesondere als Formkörper oder Rohling, die nachfolgend beschriebenen Biegefestigkeit und den E-Modul auf. Zur Definition der oben stehenden dentalen Modelle siehe Lehrbuch der Zahntechnik, Band 3, Quintessenz Verlag, A. Hohmann, W. Hielscher, 5. Aufl., 2012. Das Nachhärten bzw. Nachvergüten kann vorzugsweise z.B. mit einem Laborlichtgerät (HiLite Power 3D) oder im Lichtofen vorzugsweise mit einem Lichtspektrum von 390 - 540 nm erfolgen.

Optional kann die Zusammensetzung zusätzlich als (a.2) mindestens ein di-funktionelles Monomer, das kein Urethanacrylat oder Urethanmethacrylat ist, mindestens ein Polyetherdiacrylat, wie Poly(ethylenglycol)-Diacrylat, Poly(ethylenglycol)-Di(alkyl)acrylat, Poly(propylenglycol)-Diacrylat, Poly(propylenglycol)-Di(alkyl)acrylat oder eine Mischung von mindestens zwei der genannten Monomere enthalten. Bevorzugte Polyetherdiacrylate können ausgewählt sein aus Triethylenglycoldimethacrylat, Diethylenglycoldimethacrylat und/oder Tetraethylen-glycoldimethacrylat. Alternativ oder zusätzlich kann die Zusammensetzung Diacrylate ausgewählt aus Decandioldi(meth)acrylat, Dodecandioldi(meth)acrylat, Hexyldecandioldi(meth)acrylat, Butandioldi(meth)acrylat oder Mischungen enthaltend mindestens eines der Acrylate umfassen.

Die Bezeichnung in Klammern in den Begriffen (Methyl)acrylat oder (Alkyl)acrylat bedeutet, dass die Acrylate als Acrylat oder Methylacrylat sowie alternativ als Alkylacrylat vorliegen können.

Gleichfalls bevorzugte Monomere als mindestens ein weiteres Monomer in der Zusammensetzung können ausgewählt sein aus:
(d) mindestens einem mindestens difunktionellen Urethan(meth)acrylat, und/oder
(f) mindestens einem mono-, tri-, tetra- oder multi-funktionellen Monomer, das insbesondere kein Urethan(meth)acrylat ist.

Als mono-funktionelles Monomer kann Hydroxyethylacrylat eingesetzt werden. Gleichfalls können Hydroxypropylacrylat, Hydroxyethylmethacrylat, Hydroxypropylmethacrylat und/oder Hydroxyethylacrylat optional als Mischung von mindestens zwei der vorgenannten Monomere eingesetzt werden.

Ferner kann die Zusammensetzung (d) mindestens ein mindestens difunktionelles Urethan(meth)acrylat enthalten, das ausgewählt ist aus difunktionellen Urethan(meth)acrylaten mit bivalenter Alkylen-Gruppe (difunktionellen Urethanacrylaten und/oder difunktionellen Urethanmethacrylaten).

Das difunktionelle Urethan(meth)acrylat mit bivalenter Alkylen-Gruppe ist vorzugsweise ausgewählt aus linearen oder verzweigten mit einer bivalenten Alkylen-Gruppe funktionalisierten Urethandimethacrylaten, funktionalisierten Polyethern mit Alkylen-Gruppe(n), wie Bis(methacryloxy-2-ethoxycarbonylamino)-alkylen, Bis(methacryloxy-2-ethoxycarbonyl-amino)-substituierten Polyalkylenether, vorzugsweise 1,6-Bis(methacryloxy-2-ethoxycarbonylamino)-2,4,4-trimethylhexan, UDMA bzw. HEMA-TDMI. Bevorzugt ist ein Bis(methacryloxy-2-ethoxycarbonylamino)-alkylen, wobei Alkylen lineare oder verzweigte C3 bis C20 umfasst, vorzugsweise C3 bis C6, wie besonders bevorzugt ein mit Methyl-Gruppen substiutiertes Alkylen, wie HEMA-TMDI. Das bivalente Alkylen umfasst vorzugsweise 2,2,4-Trimethylhexamethylen und/oder 2,4,4-Trimethylhexamethylen.

Die (f) tri-, tetra- oder multi-funktionellen Monomere können ausgewählt sein aus:
Besonders bevorzugt aus Pentaerythritol-Tetraacrylat (TG 105 °C), Di-Trimethylolpropan Tetraacylat (TG 100 °C). Geeignet sind auch Trimethylolpropan-Triacrylat (TG 60 °C), Di-Pentaerythritol-Pentaacrylat (TG 90 °C), ethoxyliertes(4)Pentaerythritol-Tetraacrylat (TG 70 °C) und ethoxyliertes (4)Pentaerythritol-Tetraacrylat (TG 70 °C). Trifunktionelle Monomere können zudem ausgewählt sein aus, wobei diese vorzugsweise nur mit 0 bis 10 Gew.-%, insbesondere mit 0,01 bis 5 Gew.-% in der Gesamtzusammensetzung eingesetzt werden: Ethoxyliertes (20) Trimethylolpropan-Triacrylat (TG -40 °C), ethoxyliertes (3)Trimethylolpropan-Triacrylat, propoxyliertes (3)Triemethylolpropan-Triacrylat, ethoxyliertes (6)Trimethylolpropan-Triacrylat (TG -10 °C), ethoxyliertes (9)Trimethylolpropan-Triacrylat (TG -20 °C), propoxyliertes (3)Glyceryl-Triacrylat (TG 20 °C), ethoxyliertes (15)Trimethylolpropan-Triacrylat (TG -30 °C).

Ein TG wird vorzugsweise mittels DSC bestimmt. Die offenbarten Glasübergangstemperaturen können den dem Fachmann bekannten "Polymer Handbüchern", den Angaben der Hersteller der Monomere entnommen werden. Soweit keine Angaben zu Glasübergangstemperaturen verfügbar sind, können diese mittels DSC, DMS (Dynamisch-mechanischer Analyse), dielektrischer Relaxations-Spektroskopie oder der Dilatometrie bestimmt werden. Eine übliche Methode ist die DSC-Messung zur Bestimmung der Glasübergangstemperatur der Homopolymere. Dazu wird das Homopolymer getrocknet, auf 120 °C erhitzt, schnell auf -100 °C abgekühlt und anschließend mit 20 °C/Minute auf 150 °C oder höher auf bis zu 300 °C erhitzt und die Daten der Glasübergangstemperatur ermittelt. Gemessen wird die Glasübergangstemperatur als Mittelwert. 1/Tg = w1/Tg(1) + w2/Tg(2) mit w1 und w2 als Massenanteil der jeweiligen Co-Monomere und Tg(1) und Tg(2) für die jeweilige Glasübergangstemperatur der Homopolymere der Monomere 1 und 2. Bei weiteren CoMonomeren werden weitere Terme (wn/Tg(n)) in die Gleichung eingefügt. Die Glasüberganstemperatur von Co-Polymeren können angenähert über die Fox-Gleichung angegeben werden, siehe vorstehend und [Bulletin of the American Physical Society 1, 3 Page 123 (1956)]. TG(gesamt) kann über 1/TG(gesamt) nach der folgenden Formel ermittelt werden, wobei die Monomere gemäß folgenden Gewichtsanteilen (w1, w2, w3, w4, w5 und wn) im Polymer in der Gesamtzusammensetzung vorliegen. 1/TG(gesamt) = w1/TG₍₁₎ + w2/TG₍₂₎ + w3/TG₍₃₎+ optional w4/TG₍₄₎+optional w5/TG₍₅₎ + optional wn/TG₍ₙ₎, mit w1, w2, w3, w4, w5 und wn jeweils Gewichtsanteil des Monomers in der Gesamtzusammensetzung.

Optional können in der Zusammensetzung ein oder mehrere Füllstoffe enthalten sein, wie ein dotierter Siliziumdioxid Füllstoff, insbesondere ein Mischoxid von Zirkondioxid und Siliziumdioxid. Besonders bevorzugt sind u.a. agglomerierte Mischoxide, umfassend in Bezug auf die Gesamtzusammensetzung des Mischoxids 75 bis 99 Gew.-% Siliziumdioxid und von 1 bis 25 Gew.-% Zirkondioxid, insbesondere umfasst das Mischoxid von 85 bis 90 Gew.-% Siliziumdioxid und 10 bis 15 Gew.-% Zirkoniumdioxid, wobei es weiter bevorzugt ist, wenn die Primärteilchen der agglomerierten Oxidpartikel mikrokristalline Domänen von 4 bis 7 nm umfassen und vorteilhaft der Kristallinitätsindex 0.6 bis 0.7 - bestimmt nach der Methode von Windisch et al. (WO 01/30306A) - beträgt und die agglomerierten Oxidpartikel mit mindestens einem gegenüber mindestens einer Monomer- und/oder Polymerkomponente reaktiven organofunktionellen Silan oberflächenmodifiziert vorliegt. Diese erfindungsgemäß behandelten agglomerierten Oxidpartikel weisen hervorragende Eigenschaften in Abrasionsmessungen, bezüglich der Glanzzahl, eine hervorragende Transparenz und sehr gute Werte bei Messungen der Reflexion und Rauheit nach einem Zahnbürstentest auf.

Die Partikelgrößen der anorganischen Füllstoffe, wie des mindestens einen anorganischen Oxids, Mischoxids oder Dentalglases, bspw. umfassend Bariumaluminiumoxid, weisen für die vorliegenden Anwendungen im Durchschnitt einen mittleren Partikeldurchmesser von d₅₀ kleiner 10 µm auf, besonders bevorzugt weisen die Füllstoffe einen Partikeldurchmesser von circa 3 bis 70 nm auf, insbesondere von 10 bis 50 nm (Nanometer), optional können die Partikel aggregiert oder agglomeriert als Partikel mit bis zu 10 µm vorliegen. Die Primärpartikelgrößen der anorganischen Füllstoffe, die optional als agglomerierte und/oder aggregierte Primärpartikel vorliegen können, weisen im Durchschnitt einen Partikeldurchmesser von circa 3 bis 70 nm auf, insbesondere von 10 bis 50 nm. Bevorzugt weisen die Mischoxide von Zirkondioxid mit Siliziumdioxid eine Primärpartikelgröße von 3 bis 70 nm auf. Der Vorteil der sehr kleinen Partikeldurchmesser, die ggf. aggregiert und/oder agglomeriert vorliegen können, ist, dass das Licht an diesen Partikeln bei der Strahlenhärtung im Wesentlichen diffus gestreut wird und so zu einer verbesserten Härtung im Stereolithographie-Verfahren oder DLP-Verfahren führt.

Ferner ist es bevorzugt, wenn die Zusammensetzung nicht thixotrop ist. Zudem ist es besonders bevorzugt, wenn die Zusammensetzung eine Viskosität von kleiner 3000 m·Pas, insbesondere von 500 bis kleiner 2500 m·Pas, bevorzugt von 500 bis 2000 m·Pas, besonders bevorzugt von 500 bis 1600 m·Pas, aufweist. Die Viskosität wird vorzugsweise gemessen nach DIN 1342-2;2003-11 newtonsche Flüssigkeiten oder DIN 1342-3;2003-11 nicht newtonsche Flüssigkeiten mit einem Rheometer (Anton Par, Physika MCR 301, Viskositätsbereiche 200-3000 m·Pas bei 100/s 23 °C). Die erfindungsgemäßen Zusammensetzungen weisen keine oder vorzugsweise nur eine geringe Thixotropie auf. Die hergestellten Zusammensetzungen sind strukturviskos, wobei es bevorzugt ist, wenn die Zusammensetzungen sowohl mit als auch ohne Füllstoffe strukturviskos sind. Nach einer weiteren Ausführungsform ist es bevorzugt, dass nahezu keine Viskositätsänderungen über einen längeren Lagerzeitraum auftreten. Ferner weisen die Zusammensetzungen eine sehr gute Reaktivität bei Belichtung mit einem Laser oder DLP-Projektor auf.

Eine bevorzugte Zusammensetzung kann als Monomere ferner umfassen:
(d) mindestens ein difunktionelles Urethanacrylat oder Urethanmethacrylat ausgewählt aus difunktionellen Urethanacrylaten mit bivalenter Alkylen-Gruppe und Urethanmethacrylaten mit bivalenter Alkylen-Gruppe, und/oder
(e) mindestens ein mono-funktionelles Acrylat mit alicyclischer Gruppe und/oder mono-funktionelles Methacrylat mit alicyclischer Gruppe ausgewählt aus (Octahydro-4,7-methano-1H-indenyl)methanolacrylat, (synonym zu (Octahydro-4,7-methano-1H-indenyl)methylacrylat), (Octahydro-4,7-methano-1H-indenyl)methanolmethacrylat (TCDA), (Octahydro-4,7-methano-1H-indenyl)ethanolacrylat und (Octahydro-4,7-methano-1H-indenyl)ethanolmethacrylat, und optional,
(f) mindestens ein mono-, tri-, tetra- oder multi-funktionelles Monomer, das insbesondere kein Urethan(meth)acrylat ist. Alternativ können (i) die Monomere ferner umfassen Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern.

Bevorzugte Kombinationen der vorgenannten Monomere sind Monomere ausgewählt aus (d) in Kombination mit Monomeren aus (e) und optional zusätzlich (f). Die vorgenannten Acrylatester der Octahydro-4,7-methano-1H-indenyl)akanole können als Isomere auftreten, so dass jegliche Isomere alleine oder in Gemischen eingesetzt werden können.

Eine erfindungsgemäße Zusammensetzung umfasst (i) Monomere umfassend
(a) 5 bis 35 Gew.-% mindestens ein aus 1,3,5-Tris(m-hydroxyalkyl)isocyanurate abgeleitetes Triacrylat, wobei der Hydroxyalkyl-Rest 1 bis 8 C-Atome umfasst und linear, verzweigt und/oder cyclisch sein kann, insbesondere 1 bis 6 C-Atome, bevorzugt 1 bis 4 C-Atome, bevorzugt 1 bis 3 C-Atome, besonders bevorzugt ist Hydroxyethyl, und
(b) 5 bis 35 Gew.-% mindestens ein difunktionelles Acrylat mit bivalenter alicyclischer Gruppe und/oder difunktionelles Methacrylat mit bivalenter alicyclischer Gruppe, und
(c) 20 bis 50 Gew.-%, insbesondere 30 bis 50 Gew.-% mindestens ein disubstituiertes 4,4'-Di(oxabenzol)dialkylmethan der Formel I mit R¹, R², R⁵ und R⁶ jeweils unabhängig ausgewählt aus H oder C1 bis C4-Alkyl, und mit R³ und R⁴ jeweils bivalent C1 bis C4-Alkylen, mit n = 0 bis 6 und m = 0 bis 6, und optional
(d) 20 bis 40 Gew.-% mindestens ein mindestens difunktionelles Urethanacrylat und/oder difunktionelles Urethanmethacrylat,
(e) 5 bis 20 Gew.-%, insbesondere 5 bis 15 Gew.-%, mindestens ein monofunktionelles Acrylat mit alicyclischer Gruppe und/oder monofunktionelles Methacrylat mit alicyclischer Gruppe, und optional
(f) 1 bis 20 Gew.-% mindestens ein mono-, tri-, tetra- oder multi-funktionelles Monomer, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern,
(ii) 0,01 bis 5 Gew.-% der mindestens einen weitere Komponente umfassend mindestens einen Photoinitiator für den UV- und/oder Vis-Bereich oder ein Photoinitiatorsystem für den UV- und/oder Vis-Bereich und optional mindestens einen Stabilisator für den UV- und/oder Vis-Bereich und optional mindestens ein Pigment und/oder Farbstoff sowie weitere übliche Additive, wobei die Gesamtzusammensetzung 100 Gew.-% beträgt.

Besonders bevorzugte Photoinitiatoren umfassen alpha-Hydroxyphenylketon, Benzildimethylketal oder 2,4,6-Trimethylbenzoyldiphenylphosphinoxid, Phenyl-bis(2,4,6-trimethylbenzoyl)-Phosphinoxid, 2,4,6-Trimethylbenzoylphenylphosphinsäureethylester, und Gemische von mindestens zwei der Photoinitatoren, Phenylphosphinoxid-Kombination, Bisacylphosphinoxide (BAPO).

Typische Stabilisatoren umfassen 2,6-Di-tert.-butyl-4-methylphenol (BHT) oder Hydrochinonmonomethylether (MEHQ), 2-Hydroxy-4-methoxybenzophenon), HALS (Hindered Amine Light Stabilizers), Benzotriazol ultraviolet absorbers (UVAs) und Hydroxy Phenyl Triazines (HPT).

Nach einer weiteren bevorzugten Ausführungsform kann die Zusammensetzung umfassen:
(ii) 0,01 bis 2 Gew.-% Photoinitiator für den UV und/oder Vis-Bereich oder ein Photoinitiatorsystem für den UV und/oder Vis-Bereich, und 0,01 bis 2 Gew.-% Stabilisator, und optional
(g) 0 bis 10 Gew.-%, insbesondere 0,01 bis 7,5 Gew.-% anorganische Füllstoffe umfassend anorganische Oxide oder anorganische Mischoxide und/oder Dentalgläser, insbesondere Zirkondioxid, Mischoxide von Zirkonoxid und Siliziumdioxid, Siliziumdioxid, wobei die Gesamtzusammensetzung 100 Gew.-% beträgt.

Gleichfalls Gegenstand der Erfindung sind Zusammensetzungen, umfassend Füllstoffe mit Primärpartikelgrößen der anorganischen Füllstoffe, die optional als agglomerierte und/oder aggregierte Primärpartikel vorliegen, von im Durchschnitt mit einem Partikeldurchmesser von circa 3 bis 70 nm, insbesondere von 10 bis 50 nm. Alternativ oder zusätzlich können übliche Füllstoffe mit Partikelgrößen von 0,4 bis 10 µm in der Zusammensetzung eingesetzt werden. Ferner ist Gegenstand der Erfindung eine polymerisierte Zusammensetzung sowie ein entsprechender 3D-Formkörper als auch die nachfolgend genannten dentalen Modelle, Schienen, KFO-Apparate und prothetische Formteile oder Rohlinge, wobei die polymerisierte Zusammensetzung alternativ oder kumulativ i) a) eine Biegefestigkeit von größer gleich 75 MPa (in Anlehnung an DIN EN ISO 20795-2) aufweist und/oder b) ein E-Modul von größer gleich 2000 MPa (in Anlehnung an DIN EN ISO 20795-2) und/oder
ii) a) eine Biegefestigkeit von größer gleich 70 MPa (in Anlehnung an DIN EN ISO 20795-2), insbesondere gemessen in Wasser bei 37 °C, aufweist und/oder b) ein E-Modul von größer gleich 2000 MPa (in Anlehnung an DIN EN ISO 20795-2), insbesondere gemessen in Wasser bei 37 °C, und/oder
iii) a) eine Biegefestigkeit von größer gleich 50 MPa (in Anlehnung an DIN EN ISO 20795-2), insbesondere gemessen in Wasser bei 45 °C, aufweist und/oder b) ein E-Modul von größer gleich 1500 MPa (in Anlehnung an DIN EN ISO 20795-2), insbesondere gemessen in Wasser bei 45 °C, und/oder
iv) a) eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C, die insbesondere erhältlich sind durch Bestrahlung einer polymerisierbaren Zusammensetzung.

Dabei ist es besonders bevorzugt, wenn die polymerisierten Zusammensetzungen einen Schrumpf kleiner 7 %, vorzugsweise einen Schrumpf kleiner gleich 6,8 %, bevorzugt kleiner gleich 6,5 %, besonders bevorzugt kleiner gleich 6,0 % aufweisen (bestimmt nach Watts, Dent. Mater 7: 281-286, Okt. 1991, auch Bonded Disc-Methode genannt, bei Raumtemperatur, Translux Energy, 60 s Belichtung).

Ferner ist Gegenstand der Erfindung ein Rohling in Form eines dreidimensionalen Formkörpers einer polymerisierten Zusammensetzung geeignet zur Herstellung dentaler prothetischer Teile, orthopädischer Apparate oder dentaler Pre-Forms, wobei der Rohling a.1) eine Biegefestigkeit von größer gleich 75 MPa (in Anlehnung an DIN EN ISO 20795-2) aufweist und/oder b.1) ein E-Modul von größer gleich 2000 MPa (in Anlehnung an DIN EN ISO 20795-2) aufweist und optional
a.2) eine Biegefestigkeit von größer gleich 50 MPa (in Anlehnung an DIN EN ISO 20795-2), insbesondere gemessen in Wasser bei 45 °C, aufweist und/oder b.2) ein E-Modul von größer gleich 1500 MPa (in Anlehnung an DIN EN ISO 20795-2), insbesondere gemessen in Wasser bei 45 °C und optional
iv) a) eine Biegefestigkeit von größer gleich 40 MPa (in Anlehnung an DIN EN ISO 20795-2) insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C.

Ebenso Gegenstand der Erfindung ist die Verwendung einer Zusammensetzung zur Herstellung anatomischer Modelle, anatomischer Tischmodelle, anatomischer Modelle zur Ersetzung des dentalen Gipsmodells des Gebisses, prothetischer Teile, dentaler prothetischer Teile, orthopädischer Apparate, Alignern, Zahnschienen oder dentaler Pre-Forms, sowie die Verwendung einer erfindungsgemäßen Zusammensetzung in einem Rapid-Prototyping- oder in einem Rapid-Manufacturing- oder Rapid-Tooling-Verfahren. Bevorzugt ist eine Strahlenhärtung der Zusammensetzung mittels Laserstrahlen, LED-Leuchtmitteln oder DLP-Projektoren.

Ferner ist Gegenstand der Erfindung die Verwendung zur Herstellung dentaler prothetischer Teile umfassend Prothesenbasis oder Teile davon, künstliche Zähne, Zahnbogen mit mindestens zwei bis 16 interdental werkstoffeinstückig verbundenen künstlichen Zähnen, Kronen, provisorische Kronen, Totalprothesen, Vollkronen, Schienen für kieferorthopädische Korrekturen (ähnlich *Invisalign),* dentale Brücken, Abutments, Suprastrukturen, dentale Stege, Inlays, Onlays, orthopädische Apparate, wie Aufbissschienen, dentale Pre-Forms von künstlichen Zähnen , Bohrschablonen für die Implantologie, Mouthguards, und/oder Implantate.

Unter Dentalprodukten werden vorliegend insbesondere Dentalprodukte verstanden, die aus polymerisierbaren Zusammensetzungen herstellbar sind, wie z.B. nicht abschließend Totalprothesen, provisorische Kronen und Brücken, Inlays, Onlays, Vollkronen, Aufbissschienen, Bohrschablonen für die Implantologie, Schienen für kieferorthopädische Korrekturen (ähnlich *Invisalign),* Mouthguards, künstliche Zähne.

Zur Erfüllung hoher ästhetischer Ansprüche müssen im Dentalbereich verwendbare Zusammensetzungen zur Herstellung von definitivem Zahnersatz, wie z.B. Arbeitsmodellen, KFO Modellen, Bohrschablonen, Provisorien sowie Schienen eine hohe Transparenz aufweisen. Diese Transparenz wird in der Regel durch eine optimale Anpassung der Brechzahlen der Füllstoffe und der Polymermatrix erreicht. Hierbei sind jedoch aufgrund verschiedener physikalischer und chemischer Randbedingungen sowohl bei der Auswahl der Füllstoffe als auch der Monomere sehr enge Grenzen gesetzt.

Nach einer weiteren alternativen Ausführungsform sind polymerisierte Zusammensetzungen oder Rohlinge erhältlich, insbesondere strahlengehärtete Zusammensetzungen, insbesondere UV/Vis-gehärtete Zusammensetzungen, die vorzugsweise zusätzlich allseitig strahlengehärtet wurden, die die vorstehend genannten Eigenschaften in Bezug auf ihre Biegefestigkeit und/oder E-Modul in Anlehnung an DIN EN ISO 20795-2 aufweisen. Unter einer zusätzlich allseitigen Strahlenhärtung wird beispielsweise eine Nachvergütung im 3D-Lichtofen verstanden.

Dabei umfassen die folgenden Verfahren - Rapid-Prototyping oder Rapid-Manufacturing, ein Verfahren zur Herstellung von Werkstücken, wie ein dentales prothetisches Teil, oder Rapid-Tooling, ein Verfahren zur Herstellung von Werkzeugen - jeweils Stereolithographie-Verfahren und DLP-Verfahren. Optional kann nach dem Aushärten der polymerisierbaren Zusammensetzung in den vorgenannten Verfahren eine Nachvergütung mit UV-, Vis- oder UV/Vis-Licht erfolgen. Vorzugsweise erfolgt eine Nachvergütung der polymerisierten Zusammensetzung oder der dentalen prothetischen Teile, der orthopädischen Apparate oder dentalen Pre-Forms oder Rohlingen gleichzeitig von mindestens drei Seiten, bevorzugt von fünf bis sechs Seiten, wie dies in einem Lichtofen möglich ist. Alternativ kann die polymerisierte Zusammensetzung zusätzlich oder alternativ getempert werden.

Der Zusammensetzung können zusätzlich Farbpigmente zur Einstellung der Farbe zugesetzt werden. Zudem können der Zusammensetzung rote Fasern zugesetzt werden, um Adern der Gingiva zu imitieren. Beispielshaft geeignete Farbpigmente sind: PV Echtrot - CAS 4948-15-6, Indischblau 220943 - CAS 68186-87-8, Echtschwarz 100 - CAS 68186-91-4, Kronos 2220 - CAS 13463-67-7 und Lichtgelb 3R - CAS 68186-90-3.

In den polymerisierten Zusammensetzungen können Schichtstärken im Bereich von 5 µm, insbesondere von 25 µm bis 250 µm pro Aushärteschicht erzielt werden. Die Druckschichten liegen besonders bevorzugt bei 30 µm, 50 µm, 70 µm, 100 µm, 120 µm und 170 µm.

Eine hohe Transparenz kann durch die optimale Auswahl der Rezepturkomponenten in Bezug auf ihre Brechungsindices erreicht werden.

Als Photoinitiatoren kommen beispielsweise Benzoinalkylether oder -ester, Benzilmonoketale, Acylphosphinoxide oder aliphatische und aromatische 1,2-Diketoverbindungen, wie beispielsweise 2,2-Diethoxyacetophenon; 9,10-Phenanthrenchinon, Diacetyl, Furil, Anisil, 4,4'-Dichlorbenzil und 4,4'-Dialkoxybenzil oder Campherchinon, in Frage. Die Photoinitiatoren werden vorzugsweise zusammen mit einem Reduktionsmittel verwendet. Beispiele für Reduktionsmittel sind Amine wie aliphatische oder aromatische tertiäre Amine, beispielsweise N,N-Dimethyl-p-toluidin oder Triethanolamin, Cyanethylmethylanilin, Triethylamin, N,N-Dimethylanilin, N-Methyldiphenylamin, N,N-Dimethyl-sym.-xylidin, N,N-3,5-Tetramethylanilin und 4-Dimethylaminobenzoesäureethylester oder organische Phosphite. Gängige Photoinitiatorsysteme sind z.B. Campherchinon plus Ethyl-4-(N,N-dimethylamino)benzoat, 2-(Ethylhexyl)-4-(N,N-dimethylamino)benzoat oder N,N-Dimethylaminoethylmethacrylat.

Als Initiator für die durch UV-Licht initiierte Polymerisation eignet sich besonders 2,4,6-Trimethylbenzoyldiphenylphosphinoxid. UV-Photoinitiatoren können allein oder in Kombination mit einem Initiator für sichtbares Licht eingesetzt werden.

Besonders Photoinitiatoren und/oder Initiatorsysteme umfassen a) mindestens einen radikalischen Photoinitiator, insbesondere mindestens ein Peroxid und/oder Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid, oder ein alpha-Hydroxyketon, Campherchinon, Acylphosphinoxid. Optional können zudem Stabilisatoren zugesetzt werden und optional b) mindestens ein Co-Initiator wie ein Amin, in der Regel ein tert-Amin, insbesondere mindestens ein aromatisches Amin, wie N,N-Dimethyl-p-toluidin, N,N-Dihydroxyethyl-p-toluidin und/oder p-Dibenzylaminobenzoesäurediethylester.

Besonders bevorzugte Photoinitiatoren umfassen alpha-Hydroxyphenylketon, Benzildimethylketal oder 2,4,6-Trimethylbenzoyldiphenylphosphinoxid, Phenyl-bis(2,4,6-trimethylbenzoyl)-Phosphinoxid, 2,4,6-Trimethylbenzoylphenylphosphinsäureethylester, und Gemische von mindestens zwei der Photoinitatoren, Phenylphosphinoxid-Kombination, Bisacylphosphinoxide (BAPO).

Typische Stabilisatoren umfassen 2,6-Di-tert.-butyl-4-methylphenol (BHT) oder Hydrochinonmonomethylether (MEHQ), 2-Hydroxy-4-methoxybenzophenon), HALS (Hindered Amine Light Stabilizers), Benzotriazol ultraviolet absorbers (UVAs) und Hydroxy Phenyl Triazines (HPT).

Die Erfindung wird durch die nachgeltenden Beispiele näher erläutert.

Mit der erfindungsgemäßen Zusammensetzung können Werkstücke, Rohlinge bzw. dreidimensionale Formkörper mit sehr guter geometrischer Präzision/Auflösung gedruckt werden. Die erfindungsgemäßen Formkörper weisen sehr gute mechanische Eigenschaften selbst bei erhöhten Temperaturen auf. Ferner ist eine gute Farbstabilität bei den Werkstücken zu beobachten.

### Ausführungsbeispiele:

Allgemeines Herstellungsbeispiel: die Initiatoren werden in TCD-Acrylat oder TCD-Diacrylat vorgelöst. Anschließend werden die anderen Monomere zugegeben und die Mischung wird homogenisiert. Es können Pigmentkonzentrate oder Pigmente zugegeben werden, die Zusammensetzung wird dann vorzugsweise homogenisiert. Die hergestellte Zusammensetzung ist mit einem 3D-Drucker verarbeitbar. Hierbei ist zu beachten, dass die lichtsensiblen Initiatoren in Verbindung mit dem Umgebungslicht zu einer ungewollten Polymerisation reagieren können (die Zusammensetzung wird vorzugsweise unter geeigneten Maßnahmen in das Druckbad überführt). Belichtet wird bei 385 - 405 nm und nachgehärtet bzw. nachvergütet wird z.B. mit einem Laborlichtgerät HiLite Power 3D.

Mit der hergestellten Mischung werden auf einem 3D-Präzisionsdrucker mit der Wellenlänge 405 nm (Cara Print 4.0) Prüfkörper gemäß ISO 20795-2 (50 µm) für die nachfolgenden Abprüfungen verdruckt. Nach dem Druckprozess werden die Prüfkörper mit Isopropanol abgespült und einem Nachvergütungsprozess unterzogen. Dieser erfolgt durch beidseitiges Belichten jeweils 3 bis 5 min. oder gemäß Herstellerangaben in einer Laborlichtlampe HiLite Power 3D, 200 W (Kulzer GmbH). Eigenschaften der erfindungsgemäßen Mischung für Modell Materialien, abgeprüft nach DIN EN ISO 20795-2 bzw. angelehnt an diese Norm.

**Tabelle 1a: Thermostabilität erfindungsgemäßer Zusammensetzungen**

| | | | |
|---|---|---|---|
| | | | **Beispiel 1** |

| **Thermostabilität** | | Rechnerischer TG | 135 |
|---|---|---|---|
| **Mechanik angelehnt an** (ISO 20795-2) | | | |
| Lagerung | Messung | | |
| Lagerung trocken | Prüfung trocken | Biegefestigkeit in [MPa] | 84,2 |
| | | E-Modul in [MPa] | 2615 |
| Lagerung trocken | in Wasser (37 °C) | Biegefestigkeit in [MPa] | 81,2 |
| | | E-Modul in [MPa] | 2185 |
| Lagerung trocken | in Wasser (45 °C) | Biegefestigkeit in [MPa] | 58,2 |
| | | E-Modul in [MPa] | 1559 |
| Lagerung trocken | in Wasser (55 °C) | Biegefestigkeit in [MPa] | 44,9 |
| | | E-Modul in [MPa] | 940 |
| Schrumpf nach Watts | [%] | | 5,5 |
| Viskosität [m·Pas] | | | 1390 |

**Tabelle 1b: Erfindungsgemäße Zusammensetzungen**

| **Monomere** | TG (Homopolymer) für w1/Tg₍₁₎ bis wn/Tg₍ₙ₎ | **Beispiel 1** |
|---|---|---|
| | | Anteil in Gew.-% |
| Aliphatisches Urethandimethacrylat | 148 | 27,7 |
| ethoxyliertes (2) Bisphenol-A-Dimethacrylat | 105 | 40 |
| Tris(2-hydroxyethyl)isocyanurat-Triacrylat | 270 | 10 |
| (Octahydro-4,7-metano-1H-indenyl)methylacrylat | 35 | 10 |
| Tricyclodecan-dimethanol-diacrylat | 185 | 10 |

**Tabelle 2: Erfindungsgemäße Zusammensetzung**

| **Mischung 4** | | **MW** | **Gew.-%** |
|---|---|---|---|
| Stabilisierer | 2-Hydroxy-4-methoxybenzophenon | | 0,01 - 1,00 |
| Initiator | Phenyl-bis(2,4,6-trimethylbenzoyl)phosphinoxid | | 0,01 - 3,00 |
| Monomere | Urethandimethacrylat (Oligomer) | 700 | 10 - 30,00 |
| | Tris-(2-hydroxyethyl)- isocyanuratTriacrylat | 423,38 | 10 - 30 |
| | Ethoxyliertes Bisphenol-A-Dimethacrylat | av. 1700 | 20 - 50 |
| | (Octahydro-4,7-methano-1H-indenyl)methylacrylat | 220,31 | 10- 20 |
| | Tricyclodecandimethanoldiacrylat | 304,38 | 10 - 30 |
| Pigmente | Kronos 2056 sil. | | 0,01 - 2,0 |
| | Lichtgelb 3R | | 0,01 -1,00 |
| | Echtschwarz 100 | | 0,01 - 1,00 |
| | rot 100 | | 0,01 - 1,00 |

| | |
|---|---|
| Total: | 100,00 |

**Tabelle 3: Thermostabilität Zusammensetzungen Dritter Thermostabilität**

| | | **Mechanik** angelehnt an ISO 20795-2 | Dreve Fotodent | Detax Free Print | B9 Creations B9R - 5 |
|---|---|---|---|---|---|
| Lagerung | Prüfung | | | | |
| Lagerung trocken | trocken | Biegefestigkeit in [MPa] | 73,2 | 74,9 | 46,9 |
| | | E-Modul in [MPa] | 1991 | 1697 | 983 |
| Lagerung trocken | in Wasser (37 °C) | Biegefestigkeit in [MPa] | 65,8 | 35 | 11,1 |
| | | E-Modul in [MPa] | 1594 | 557 | F max - 18 |
| Lagerung trocken | in Wasser (45 °C) | Biegefestigkeit in [MPa] | 54 | nicht möglich | nicht möglich |
| | | E-Modul in [MPa] | 1288 | | |
| Lagerung trocken | in Wasser (55°C) | Biegefestigkeit in [MPa] | 40,9 | nicht möglich | nicht möglich |
| | | E-Modul in [MPa] | 914 | | |
| Schrumpf nach Watts | | [%] | 5,83 | 7,7 | 7,2 |

## Patentansprüche

1. Polymerisierbare, strahlenhärtbare Zusammensetzung umfassend
(i) Monomere und
(ii) mindestens eine weitere Komponente,
**dadurch gekennzeichnet, dass**
(i) die Monomere umfassen
(a) 5 bis 35 Gew.-% mindestens ein aus 1,3,5-Tris(ω-hydroxyalkyl)isocyanurate abgeleitetes Triacrylat, wobei der Hydroxyalkyl-Rest 1 bis 8 C-Atome umfasst, und
(b) 5 bis 35 Gew.-% mindestens ein difunktionelles Acrylat mit bivalenter alicyclischer Gruppe und/oder difunktionelles Methacrylat mit bivalenter alicyclischer Gruppe, und
(c) 20 bis 50 Gew.-% mindestens ein disubstituiertes 4,4'-Di(oxabenzol)dialkylmethan der Formel I 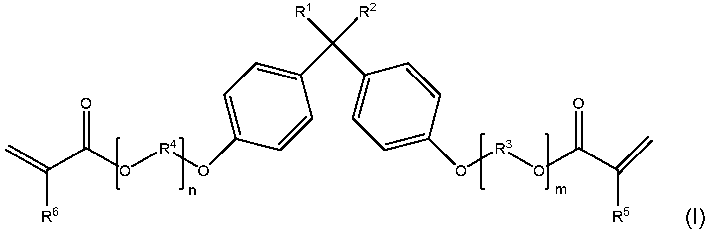 mit R¹, R², R⁵ und R⁶ jeweils unabhängig ausgewählt aus H oder C1 bis C4-Alkyl, und mit R³ und R⁴ jeweils bivalent C1 bis C4-Alkylen, mit n = 0 bis 6 und m = 0 bis 6, und
(d) 20 bis 40 Gew.-% mindestens ein mindestens difunktionelles Urethanacrylat und/oder difunktionelles Urethanmethacrylat,
(e) 5 bis 20 Gew.-% mindestens ein monofunktionelles Acrylat mit alicyclischer Gruppe und/oder monofunktionelles Methacrylat mit alicyclischer Gruppe, und optional
(f) 1 bis 20 Gew.-% mindestens ein mono-, tri-, tetra- oder multi-funktionelles Monomer, insbesondere Acrylatester und/oder Methacrylatester von Polyethern ausgewählt aus Di-Methacrylestern von Polyethern, tri-, tetra oder multifunktionellen Methacrylestern von Polyethern, Di-Acrylestern von Polyethern, tri-, tetra und/oder multifunktionellen Acrylestern von Polyethern,
(ii) 0,01 Gew.-% bis 5 Gew.-% der mindestens einen weiteren Komponente umfassend mindestens einen Photoinitiator für den UV- und/oder Vis-Bereich oder ein Photoinitiatorsystem für den UV- und/oder Vis-Bereich und optional mindestens einen Stabilisator für den UV- und/oder Vis-Bereich und optional mindestens ein Pigment und/oder Farbstoff sowie weitere übliche Additive,
wobei die Gesamtzusammensetzung 100 Gew.-% beträgt.

2. Zusammensetzung nach Anspruch 1, **dadurch gekennzeichnet, dass** die Viskosität der Zusammensetzung bei Raumtemperatur ca. 20 °C bis 23 °C kleiner gleich 3000 m·Pas beträgt, wobei die Viskosität gemessen wird wie in der Beschreibung offenbart ist, insbesondere von 500 bis kleiner 2500 m·Pas.

3. Zusammensetzung nach Anspruch 1, **dadurch gekennzeichnet, dass** (a.1) das mindestens eine aus 1,3,5-Tris(ω-hydroxyalkyl)isocyanurat abgeleitete Triacrylat umfasst 1,3,5-Tris(2-hydroxyethyl)isocyanurat-Triacrylat, 1,3,5-Tris(2-hydroxymethyl)isocyanurat-Triacrylat, 1,3,5-Tris(2-hydroxyethyl)isocyanurat-Trimethacrylat, 1,3,5-Tris(2-hydroxymethyl)isocyanurat-Trimethacrylat oder Mischungen enthaltend mindestens zwei der Monomere.

4. Zusammensetzung nach einem der Ansprüche 1 bis 3, **dadurch gekennzeichnet, dass** das mindestens eine di-funktionelle Monomer, das kein Urethan(meth)acrylat ist ausgewählt ist aus (b) difunktionellen Acrylaten mit bivalenter alicyclischer Gruppe und difunktionellen Methacrylaten mit bivalenter alicyclischer Gruppe.

5. Zusammensetzung nach Anspruch 3 oder 4, **dadurch gekennzeichnet, dass** (b) ausgewählt ist aus Tricyclodecandimethanoldiacrylat (TCDDA), Tricyclodecandimethanoldimethacrylat, Tricyclodecandiethanoldiacrylat, Tricyclodecandiethanoldimethacrylat und/oder Mischungen dieser.

6. Zusammensetzung nach einem der Ansprüche 1 bis 3, **dadurch gekennzeichnet, dass** das (a.2) mindestens eine di-funktionelle Monomer, das kein Urethan(meth)acrylat ist, umfasst (c) mindestens ein disubstituiertes 4,4'-Di(oxabenzol)dialkylmethan der Formel I 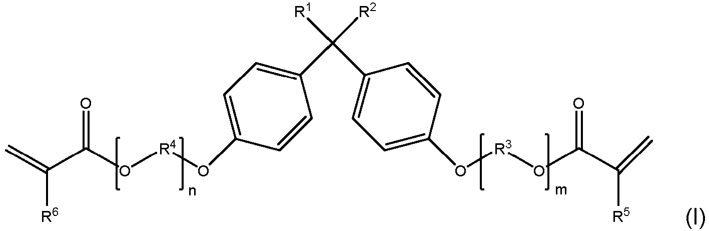 mit R¹ und R² jeweils Methyl-, und R⁵ und R⁶ gleich und ausgewählt aus H, Methyl und Ethyl, insbesondere mit R⁵ und R⁶ gleich und ausgewählt aus H und Methyl, und mit R³ und R⁴jeweils unabhängig bivalent Ethylen oder Propylen mit n = 1 bis 6, bevorzugt n = 2 bis 4, und mit m = 1 bis 6, bevorzugt m = 2 bis 4, bevorzugt mit n = 2 und m = 2 oder mit n = 4 und m = 4 sowie Mischungen dieser.

7. Zusammensetzung nach Anspruch 3, **dadurch gekennzeichnet, dass** das
(d) mindestens eine difunktionelle Urethanacrylat oder Urethanmethacrylat ausgewählt ist aus difunktionellen Urethanacrylaten mit bivalenter Alkylen-Gruppe und Urethanmethacrylaten mit bivalenter Alkylen-Gruppe, und/oder
(e) mindestens eine monofunktionelle Acrylat mit alicyclischer Gruppe und/oder monofunktionelles Methacrylat mit alicyclischer Gruppe ausgewählt ist aus (Octahydro-4,7-methano-1H-indenyl)methanolacrylat, (Octahydro-4,7-methano-1H-indenyl)methanolmethacrylat, (Octahydro-4,7-methano-1H-indenyl)ethanolacrylat und (Octahydro-4,7-methano-1H-indenyl)ethanolmethacrylat und Gemischen enthaltend diese, und optional
(f) mindestens ein mono-, tri-, tetra- oder multi-funktionelles Monomer, das insbesondere kein Urethan(meth)acrylat ist.

8. Polymerisierte Zusammensetzung nach einem der Ansprüche 1 bis 7, **dadurch gekennzeichnet, dass** die polymerisierte Zusammensetzung alternativ oder kumulativ
i) a) eine Biegefestigkeit von größer gleich 75 MPa (in Anlehnung an DIN EN ISO 20795-2), aufweist und/oder b) ein E-Modul von größer gleich 2000 MPa (in Anlehnung an DIN EN ISO 20795-2), und/oder
ii) a) eine Biegefestigkeit von größer gleich 70 MPa (in Anlehnung an DIN EN ISO 20795-2), insbesondere gemessen in Wasser bei 37 °C, aufweist und/oder b) ein E-Modul von größer gleich 2000 MPa (in Anlehnung an DIN EN ISO 20795-2), insbesondere gemessen in Wasser bei 37 °C aufweist, und/oder
iii) a) eine Biegefestigkeit von größer gleich 50 MPa (in Anlehnung an DIN EN ISO 20795-2), insbesondere gemessen in Wasser bei 45 °C, aufweist und/oder b) ein E-Modul von größer gleich 1500 MPa (in Anlehnung an DIN EN ISO 20795-2), insbesondere gemessen in Wasser bei 45 °C aufweist, und/oder
iv) a) eine Biegefestigkeit von größer gleich 40 MPa (in Anlehnung an DIN EN ISO 20795-2), insbesondere gemessen in Wasser bei 55 °C, aufweist und/oder b) ein E-Modul von größer gleich 900 MPa (in Anlehnung an DIN EN ISO 20795-2), insbesondere gemessen in Wasser bei 55 °C aufweist.

9. Rohling in Form eines dreidimensionalen Formkörpers einer polymerisierten Zusammensetzung nach einem der Ansprüche 1 bis 8 zur Herstellung dentaler prothetischer Teile, orthopädischer Apparate oder dentaler Pre-Forms, **dadurch gekennzeichnet, dass** der Rohling a.1) eine Biegefestigkeit von größer gleich 75 MPa (in Anlehnung an DIN EN ISO 20795-2) aufweist und/oder b.1) ein E-Modul von größer gleich 2000 MPa (in Anlehnung an DIN EN ISO 20795-2) aufweist und optional a.2) eine Biegefestigkeit von größer gleich 50 MPa (in Anlehnung an DIN EN ISO 20795-2)-2, insbesondere gemessen in Wasser bei 45 °C, aufweist und/oder b.2) ein E-Modul von größer gleich 1500 MPa (in Anlehnung an DIN EN ISO 20795-2) aufweist, insbesondere gemessen in Wasser bei 45 °C.

10. Verwendung einer Zusammensetzung nach einem der Ansprüche 1 bis 9 zur Herstellung anatomischer Modelle, anatomischer Tischmodelle, dentaler Arbeitsmodelle, dentaler Vollmodelle, dentaler Stumpfmodelle, anatomischer oder dentaler Sägeschnittmodelle, insbesondere Situationsmodelle, Gegenbissmodelle, Funktionsmodelle, Vormodelle, Reparaturmodelle, Präzisionsmodelle, anatomische Modelle zur Ersetzung des dentalen Gipsmodells des Gebisses, Alignern, Zahnschienen, prothetischer Teile, dentaler prothetischer Teile, orthopädischer Apparate oder dentaler Pre-Forms, insbesondere einer Zusammensetzung nach einem der Ansprüche 1 bis 9, in einem Rapid-Prototyping- oder in einem Rapid-Manufacturing- oder Rapid-Tooling-Verfahren.

11. Verwendung nach Anspruch 10, **dadurch gekennzeichnet, dass** die dentalen prothetischen Teile umfassen Prothesenbasis oder Teile davon, künstliche Zähne, Zahnbogen mit mindestens zwei bis 16 interdental werkstoffeinstückig verbundenen künstlichen Zähnen, Kronen, provisorische Kronen, Totalprothesen, Vollkronen, Schienen für kieferorthopädische Korrekturen (ähnlich Invisalign), dentale Brücken, Abutments, Suprastrukturen, dentale Stege, Inlays, Onlays, orthopädische Apparate, wie Aufbissschienen, dentale Pre-Forms von künstlichen Zähnen, Bohrschablonen für die Implantologie, Mouthguards, und/oder Implantate.

## Claims

1. Polymerisable radiation-curable composition comprising
(i) monomers and
(ii) and least one further component,
characterised that,
(i) monomers comprising
(a) 5 to 35 % by weight at least one triacrylate derived from 1,3,5-tris(ω-hydroxyalkyl) isocyanurate, wherein the hydroxyalkyl residue comprises 1 to 8 C-atoms, and
(b) 5 to 35 % by weight at least one di-functional acrylate having a bivalent alicyclic group and/or di-functional methacrylate having a bivalent alicyclic group, and
(c) 20 to 50 % by weight at least one disubstituted 4,4'-di(oxabenzol) dialkyl methane of formula I 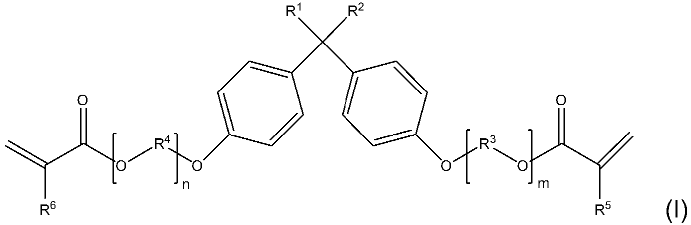 with R¹, R², R⁵ and R⁶ each independently selected from H or C1 to C4-alkyl, and with R³ and R⁴ each bivalent C1 to C4-alkylene, with n = 0 to 6 and m = 0 to 6, and optionally
(d) 20 to 40 % by weight at least one at least di-functional urethane acrylate and/or di-functional urethane methacrylate,
(e) 5 to 20 % by weight at least one mono-functional acrylate having an alicyclic group and/or mono-functional methacrylate having an alicyclic group, and optinally
(f) 1 to 20 % by weight at least one mono-, tri-, tetra- or multi-functional monomer, in particular acrylic esters and/or methacrylic esters of polyethers selected from dimethacrylic esters of polyethers, tri-, tetra- or multi-functional methacrylic esters of polyethers, diacrylic esters of polyethers, tri-, tetra- and/or multi-functional acrylic esters of polyethers,
(ii) 0.01 % by weight to 5 % by weight of the at least one further component comprising at least one photo-initiator for the UV and/or Vis spectral region or a photo-initiator system for the UV and/or Vis spectral region, and optionally at least one stabiliser for the UV- and/or Vis spectral region, and optionally at least one pigment and/or dye as well as further usual additives,
wherein the total composition amounts to 100 % by weight.

2. Composition according to claim 1, wherein the viscosity of the composition at ambient room temperature (approx. 20 °C to 23 °C) is less than or equal to 3000 m·Pas, wherein the viscosity will be measured according to the disclosure in the description, in particular from 500 to 2500 m·Pas.

3. Composition according to claim 1, wherein
(a.1) the at least one triacrylate derived from 1,3,5-tris(ω-hydroxyalkyl) isocyanurate comprises 1,3,5-tris(2-hydroxyethyl) isocyanurate triacrylate, 1,3,5-tris(2-hydroxymethyl) isocyanurate triacrylate, 1,3,5-tris(2-hydroxyethyl) isocyanurate trimethacrylate, 1,3,5-tris(2-hydroxymethyl) isocyanurate trimethacrylate or mixtures containing at least two of the monomers.

4. Composition according any of claims 1 to 3, wherein the at least one di-functional monomer, not being a urethane (meth)acrylate, is selected from (b) di-functional acrylates having a bivalent alicyclic group and di-functional methacrylates having a bivalent alicyclic group.

5. Composition according to claim 3 or 4, wherein (b) is selected from tricyclodecane dimethanol diacrylate (TCDDA), tricyclodecane dimethanol dimethacrylate, tricyclodecane diethanol diacrylate, tricyclodecane diethanol dimethacrylate and/or mixtures thereof.

6. Composition according to any of claims 1 to 3, wherein the (a.2) at least one di-functional monomer, not being a urethane (methacrylate), comprises (c) at least one disubstituted 4,4'-di(oxabenzol) dialkyl methane of formula I 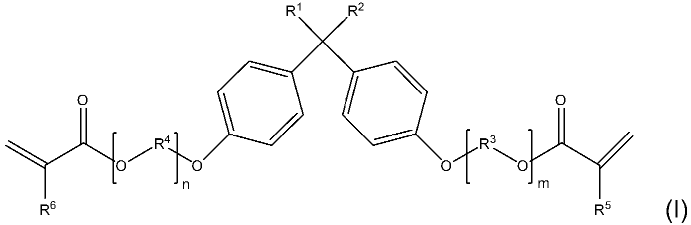 with R¹ and R² each methyl, and R⁵ and R⁶ the same and selected from H, methyl and ethyl, and with R³ and R⁴ each independently bivalent ethylene or propylene, with n = 1 to 6, preferred n = 2 to 4, and m = 1 to 6, preferred m = 2 to 4, preferred n = 2 and m = 2 or n = 4 and m = 4, as well as mixtures thereof.

7. Composition according to claim 3, wherein the
(d) at least one di-functional urethane acrylate or urethane methacrylate is selected from di-functional urethane acrylates having a bivalent alkylene group and urethane methacrylates having a bivalent alkylene group, and/or
(e) at least one mono-functional acrylate having an alicyclic group and/or mono-functional methacrylate having an alicyclic group is selected from (octahydro-4,7-methano-1H-indenyl) methanol acrylate, (octahydro-4,7-methano-1H-indenyl) methanol methacrylate, (octahydro-4,7-methano-1H-indenyl) ethanol acrylate and (octahydro-4,7-methano-1H-indenyl) ethanol methacrylate and mixtures containing them, and optionally
(f) at least one mono-, tri-, tetra- or multi-functional monomer, which in particular is not an urethane (methacrylate).

8. Polymerised composition according to claim 1, wherein the polymerised composition has, alternatively or cumulatively,
i) a) a flexural strength of greater than or equal to 75 MPa (following DIN EN ISO 20795-2), and/or b) an E-modulus of greater than or equal to 2000 MPa (following DIN EN ISO 20795-2), and/or
ii) a) a flexural strength of greater than or equal to 70 MPa (following DIN EN ISO 20795-2), measured in water at 37 °C, and/or b) an E-modulus of greater than or equal to 2000 MPa (following DIN EN ISO 20795-2), measured in water at 37 °C, and/or
iii) a) a flexural strength of greater than or equal to 50 MPa (following DIN EN ISO 20795-2), measured in water at 45 °C, and/or b) an E-modulus of greater than or equal to 1500 MPa (following DIN EN ISO 20795-2), measured in water at 45 °C, and/or
iv) a) a flexural strength of greater than or equal to 40 MPa (following DIN EN ISO 20795-2), measured in water at 55 °C, and/or b) an E-modulus of greater than or equal to 900 MPa (following DIN EN ISO 20795-2), measured in water at 55 °C.

9. Blank in the form of a three-dimensional moulded body of a polymerised composition according to claim 1 for producing dental prosthetic parts, orthopaedic appliances or dental pre-forms, wherein the blank has a.1) a flexural strength of greater than or equal to 75 MPa (following DIN EN ISO 20795-2), and/or b.1) an E-modulus of greater than or equal to 2000 MPa (following DIN EN ISO 20795-2), and optionally
a.2) a flexural strength of greater than or equal to 50 MPa (following DIN EN ISO 20795-2), measured in water at 45 °C, and/or b.2) an E-modulus of greater than or equal to 1500 MPa (following DIN EN ISO 20795-2), measured in water at 45 °C.

10. Use of a composition according to any of claims 1 to 9 for producing anatomical models, anatomical table-top models, dental working models, dental full models, dental die models, anatomical or dental saw-cut models, situation models, counter-bite models, functional models, Pre-models, repair models, precision models, anatomical models for replacement of the dental plaster model of the dentition, aligners, dental splints, prosthetic pars, dental prosthetic parts, orthopaedic appliances or dental pre-forms, in a rapid prototyping or in a rapid manufacturing or rapid tooling method.

11. Use according to claim 10, wherein the dental prosthetic parts comprise prosthesis base or parts thereof, artificial teeth, dental arch having at least two to 16 artificial teeth being interdentally connected in an integral manner, crowns, provisional crowns, total prostheses, total crowns, splints for orthodontic corrections, dental bridges, abutments, suprastructures, dental bars, inlays, onlays, orthopaedic appliances including occlusal splints, dental pre-forms of artificial teeth, surgical guides for implantology, mouthguards, and/or implants.

## Revendications

1. Composition polymérisable et durcissable par rayonnement, comprenant
(i) des monomères et
(ii) au moins un autre composant,
**caractérisée en ce que**
(i) les monomères comprennent
(a) de 5à 35 % en poids d'au moins un triacrylate dérivé du 1,3,5-tris(ω-hydroxyalkyl)isocyanurate, le radical hydroxyalkyle comportant de 1 à 8 atomes de carbone, et
(b) de 5 à 35 % en poids d'au moins un acrylate difonctionnel comportant un groupe alcicyclique bivalent et/ou un méthacrylate difonctionnel comportant un groupe alcicyclique bivalent, et
(c) de 20 à 50 % en poids d'au moins un 4,4'-di(oxabenzol)dialkylméthane disubstitué de formule I 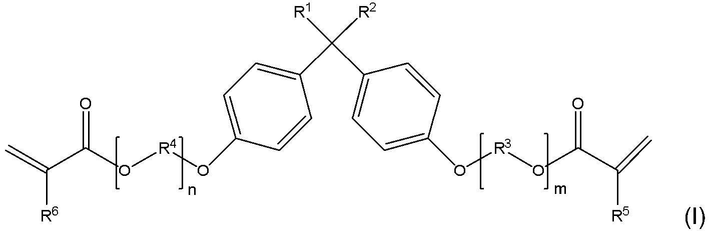 R¹, R², R⁵ et R⁶ étant chacun choisis indépendamment parmi H et un groupe alkyle en C1 à C4, et R³ et R⁴ représentant chacun un groupe alkylène bivalent en C1 à C4, avec n = 0 à 6 et m = 0 à 6, et
(d) de 20 à 40 % en poids d'au moins un uréthane-acrylate au moins difonctionnel et/ou un uréthane-méthacrylate difonctionnel,
(e) de 5 à 20 % en poids d'au moins un acrylate monofonctionnel comportant un groupe alcicyclique et/ou un méthacrylate monofonctionnel comportant un groupe alcicyclique, et facultativement,
(f) de 1 à 20 % en poids d'au moins un monomère monofonctionnel, trifonctionnel, tétrafonctionnel ou multifonctionnel, notamment un ester acrylate et/ou un ester méthacrylate de polyéthers, choisi parmi les diesters méthacryliques de polyéthers, les esters méthacryliques trifonctionnels, tétrafonctionnels ou multifonctionnels de polyéthers, les diesters acryliques de polyéthers, ainsi que les esters acryliques trifonctionnels, tétrafonctionnels et/ou multifonctionnels de polyéthers,
(ii) de 0,01 % en poids à 5 % en poids dudit au moins un autre composant, comprenant au moins un photoinitiateur pour le domaine UV et/ou visible, ou un système photoinitiateur pour le domaine UV et/ou visible, et facultativement, au moins un stabilisant pour le domaine UV et/ou visible, et facultativement, au moins un pigment et/ou colorant, ainsi que d'autres additifs usuels,
la composition totale représentant 100 % en poids.

2. Composition selon la revendication 1, **caractérisée en ce que** la viscosité de la composition à température ambiante, comprise entre environ 20 °C et 23 °C, est inférieure ou égale à 3000 m·Pas, la viscosité étant mesurée selon la méthode décrite dans la description, et étant notamment comprise entre 500 et moins de 2500 m·Pas.

3. Composition selon la revendication 1, **caractérisée en ce que**
(a.1) ledit au moins un triacrylate dérivé du 1,3,5-tris(ω-hydroxyalkyl)isocyanurate comprend le triacrylate de 1,3,5-tris(2-hydroxyéthyl)isocyanurate, le triacrylate de 1,3,5-tris(2-hydroxyméthyl)isocyanurate, le triméthacrylate de 1,3,5-tris(2-hydroxyéthyl)isocyanurate, le triméthacrylate de 1,3,5-tris(2-hydroxyméthyl)isocyanurate ou des mélanges contenant au moins deux de ces monomères.

4. Composition selon l'une des revendications 1 à 3, **caractérisée en ce que** ledit au moins un monomère difonctionnel, qui n'est pas un uréthane(méth)acrylate, est choisi parmi (b) les acrylates difonctionnels comportant un groupe alcicyclique bivalent et les méthacrylates difonctionnels comportant un groupe alcicyclique bivalent.

5. Composition selon la revendication 3 ou 4, **caractérisée en ce que** (b) est choisi parmi le diacrylate de tricyclodécanediméthanol (TCDDA), le diméthacrylate de tricyclodécanediméthanol, le diacrylate de tricyclodécanediéthanol, le diméthacrylate de tricyclodécanediéthanol et/ou des mélanges de ceux-ci.

6. Composition selon l'une des revendications 1 à 3, **caractérisée en ce que** ledit (a.2) au moins un monomère difonctionnel, qui n'est pas un uréthane(méth)acrylate, comprend
(c) au moins un 4,4'-di(oxabenzol)dialkylméthane disubstitué de formule I 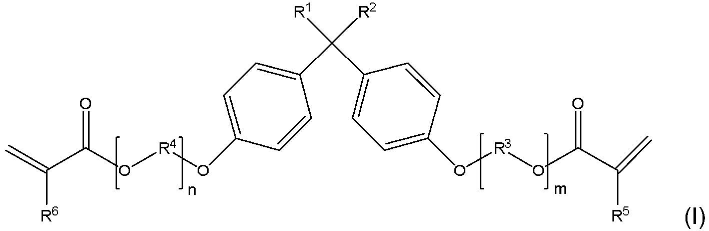 R¹et R² représentant chacun un groupe méthyle, et R⁵ et R⁶ étant identiques et choisis parmi H, méthyle et éthyle, en particulier R⁵ et R⁶ étant identiques et choisis parmi H et méthyle, et R³ et R⁴ représentant chacun indépendamment un groupe éthylène ou propylène bivalent, avec n = 1 à 6, de préférence n = 2 à 4, et avec m = 1 à 6, plus particulièrement m = 2 à 4, plus particulièrement avec n = 2 et m = 2 ou avec n = 4 et m = 4, ainsi que des mélanges de ces composés.

7. Composition selon la revendication 3, **caractérisée en ce que**
(d) ledit au moins un uréthane-acrylate difonctionnel ou uréthane-méthacrylate difonctionnel est choisi parmi les uréthane-acrylates difonctionnels comportant un groupe alkylène bivalent et les uréthane-méthacrylates comportant un groupe alkylène bivalent, et/ou
(e) ledit au moins un acrylate monofonctionnel comportant un groupe alcicyclique et/ou méthacrylate monofonctionnel comportant un groupe alcicyclique est choisi parmi l'acrylate de (octahydro-4,7-méthano-1H-indényl)méthanol, le méthacrylate de (octahydro-4,7-méthano-1H-indényl)méthanol, l'acrylate de (octahydro-4,7-méthano-1H-indényl)éthanol et le méthacrylate de (octahydro-4,7-méthano-1H-indényl)éthanol, ainsi que les mélanges contenant ces composés, et facultativement,
(f) ledit au moins un monomère monofonctionnel, trifonctionnel, tétrafonctionnel ou multifonctionnel, qui n'est en particulier pas un uréthane(méth)acrylate.

8. Composition polymérisée selon l'une des revendications 1 à 7, **caractérisée en ce que** la composition polymérisée présente, alternativement ou cumulativement,
i) a) une résistance à la flexion supérieure ou égale à 75 MPa (conformément à la norme DIN EN ISO 20795-2) et/ou b) un module d'élasticité supérieur ou égal à 2000 MPa (conformément à la norme DIN EN ISO 20795-2), et/ou
ii) a) une résistance à la flexion supérieure ou égale à 70 MPa (conformément à la norme DIN EN ISO 20795-2), en particulier mesurée dans l'eau à 37 °C, et/ou b) un module d'élasticité supérieur ou égal à 2000 MPa (conformément à la norme DIN EN ISO 20795-2), en particulier mesuré dans l'eau à 37 °C, et/ou
iii) a) une résistance à la flexion supérieure ou égale à 50 MPa (conformément à la norme DIN EN ISO 20795-2), en particulier mesurée dans l'eau à 45 °C, et/ou b) un module d'élasticité supérieur ou égal à 1500 MPa (conformément à la norme DIN EN ISO 20795-2), en particulier mesuré dans l'eau à 45 °C, et/ou
iv) a) une résistance à la flexion supérieure ou égale à 40 MPa (conformément à la norme DIN EN ISO 20795-2), en particulier mesurée dans l'eau à 55 °C, et/ou b) un module d'élasticité supérieur ou égal à 900 MPa (conformément à la norme DIN EN ISO 20795-2), en particulier mesuré dans l'eau à 55 °C.

9. Ébauche sous la forme d'un corps tridimensionnel moulé, constitué d'une composition polymérisée selon l'une des revendications 1 à 8, destinée à la fabrication de pièces prothétiques dentaires, d'appareils orthopédiques ou de préformes dentaires, **caractérisée en ce que** l'ébauche présente a.1) une résistance à la flexion supérieure ou égale à 75 MPa (conformément à la norme DIN EN ISO 20795-2) et/ou b.1) un module d'élasticité supérieur ou égal à 2 000 MPa (conformément à la norme DIN EN ISO 20795-2), et facultativement, a.2) une résistance à la flexion supérieure ou égale à 50 MPa (conformément à la norme DIN EN ISO 20795-2), en particulier mesurée dans l'eau à 45 °C, et/ou b.2) un module d'élasticité supérieur ou égal à 1 500 MPa (conformément à la norme DIN EN ISO 20795-2), en particulier mesuré dans l'eau à 45 °C.

10. Utilisation d'une composition selon l'une des revendications 1 à 9 pour la fabrication de modèles anatomiques, de modèles anatomiques de table, de modèles de travail dentaires, de modèles dentaires pleins, de modèles de moignons dentaires, de modèles anatomiques ou dentaires en coupe, notamment de modèles de situation, de modèles d'antagonistes, de modèles fonctionnels, de modèles préliminaires, de modèles de réparation, de modèles de précision, de modèles anatomiques destinés à remplacer le modèle en plâtre de la dentition, d'aligneurs, de gouttières dentaires, de pièces prothétiques, de pièces prothétiques dentaires, d'appareils orthopédiques ou de préformes dentaires, notamment d'une composition selon l'une des revendications 1 à 9, dans le cadre d'un procédé de prototypage rapide, de fabrication rapide ou d'outillage rapide.

11. Utilisation selon la revendication 10, **caractérisée en ce que** les pièces prothétiques dentaires comprennent des bases de prothèses ou des parties de celles-ci, des dents artificielles, des arcades dentaires comportant au moins 2 à 16 dents artificielles reliées entre elles d'une seule pièce par un matériau interdentaire, des couronnes, des couronnes provisoires, des prothèses complètes, des couronnes intégrales, des gouttières destinées aux corrections orthodontiques (similaires à Invisalign), des bridges dentaires, des piliers implantaires, des suprastructures, des barres dentaires, des inlays, des onlays, des appareils orthopédiques tels que des gouttières occlusales, des préformes dentaires de dents artificielles, des guides chirurgicaux pour l'implantologie, des protège-dents et/ou des implants.
